# EUROPÄISCHE PATENTSCHRIFT

(11) **EP 0 836 848 B1**
(45) Veröffentlichungstag und Bekanntmachung des Hinweises auf die Patenterteilung: **26.11.2003**
(21) Anmeldenummer: 97250303.1
(22) Anmeldetag: 16.10.1997
(51) Int. Cl.: A61K 7/42

(54) **Kosmetisches Hautbräunungs- und Lichtschutzmittel**
Cosmetic tanning and sunscreen composition
Composition cosmétique pour le bronzage de la peau et pour la photoprotection

(30) Priorität: 17.10.1996 DE 19644637
(43) Veröffentlichungstag der Anmeldung: 22.04.1998
(73) Patentinhaber: LANCASTER GROUP GmbH, 55116 Mainz (DE)
(72) Erfinder: Cernasov, Domnica, Ringwood, NJ 07556 (US); Macchio, Ralph, Flanders, NJ 07836 (US); Stanzl, Klaus, Dr., White Plains, N.Y. 10605 (US); Zastrow, Leonhard, Prof. Dr., 98000 Monaco (MC)
(74) Vertreter: Walter, Wolf-Jürgen

(56) Entgegenhaltungen:
- EP-A- 0 281 394
- WO-A-94/15580
- WO-A-95/34278
- WO-A-96/28136
- PATENT ABSTRACTS OF JAPAN vol. 018, no. 547 (C-1262), 19. Oktober 1994 (1994-10-19) & JP 06 192610 A (PENTEL KK), 12. Juli 1994 (1994-07-12)
- PATENT ABSTRACTS OF JAPAN vol. 1995, no. 08, 29. September 1995 (1995-09-29) & JP 07 133210 A (KAO CORP), 23. Mai 1995 (1995-05-23)

## Beschreibung

Die Erfindung betrifft ein neues kosmetisches Selbstbräunungsmittel mit zugleich lichtschützender Wirkung.

Bei der Herstellung von kosmetischen Präparaten, die sowohl einen Selbstbräunungseffekt auf der Haut herbeiführen als auch eine Lichtschutzwirkung haben, kommt es auf eine ausgewogenen Zusammensetzung an, um eine stabile Emulsion zu erhalten, da einzelne Komponenten mit oftmals entgegengesetzten Eigenschaften in eine solche Emulsion integriert werden müssen.

Aus der US-A-4 193 989 ist ein wasserbeständiges Selbstbräunungs-Gel mit Lichtschutzwirkung bekannt, das aus 80 - 90 % eines alkoholischen Lösungsmittels, 1-12 % UV-Absorber, der in dem Lösungsmittel löslich ist, 3 bis 7 % Erweichungsmittel, 1 bis 2 % Gelierungsmittel für das Lösungsmittel und 1 bis 3 % einer Säure besteht. Das Gelierungsmittel ist Hydroxypropylcelluloseacetat, Hyxdroxypropylstärkeacetat oder Gemische davon und bewirkt eine festere Bindung des Gel-Films auf der Haut.

Die DE-A-43 42 719 beschreibt kosmetische oder dermatologische O/W-Emulsionen, die Gemische aus hydrophoben anorganischen Pigmenten und Aminosäuren enthalten, wobei mit diesen Emulsionen ein "stinging" der Haut herabgesetzt werden soll. Die Emulsionen können auch verschiedene UVA- und UVB-Filter sowie Pre-Soleil- und Aprés-Soleil-Produkte enthalten.

Der Erfindung liegt die Aufgabe zugrunde, unter Verwendung anorganischer Pigmente eine stabile O/W-Emulsion eines kosmetischen Hautbräunungs- und Lichtschutzmittel bereitzustellen, das bei mittlerer Lichtschutzwirkung zugleich sehr gute Feuchhalteeigenschaften ohne wesentliche Feuchthaltemittelzusätze und sehr gute Wasserabweisungseigenschaften des auf der Haut aufgetragenen Filmes hat.

Erfindungsgemäß bereitgestellt wird ein kosmetisches Hautbräunungs- und Lichtschutzmittel, gekennzeichnet durch eine O/W-Emulsion, bestehend aus (bezogen auf das Gesamtgewicht der Zusammensetzung)
(a) mit Perfluoralkylphosphaten behandelten anorganischen Pigmenten., wobei die Pigmente aus der Gruppe ausgewählt sind, die aus farbgebenden Eisenoxiden, Titandioxid und deren Gemischen besteht, in einem Anteil von 5 bis 14 Gew-%;
(b) Dispergiermitteln der Gruppe, die wenigstens aus (b1) Cetyl Dimethicone Copolyol und (b2) Cetyl Dimethicone besteht, in einem Anteil im Bereich von 2 bis 10 Gew-% und mit einem Verhältnis von (b1) zu (b2) im Bereich von 15 bis 3 zu 40 bis 8; und
(c) weiteren bekannten Träger- und Hilfsstoffen sowie gegebenenfalls Dispergiermitteln, wobei der Anteil an zugesetzten Feuchthaltemitteln unter 4 Gew-% liegt.

Die Pigmente liegen in einer Form vor, bei der sie durch Behandlung mit einem Perfluoralkylphosphat, z.B. mit dem Perfluoralkylphosphat-Diethanolaminsalz im sauren Medium und unter Erwärmung in eine Form umgewandelt wurden, bei der die Pigmentteilchen-Oberfläche mit der Perfluorverbindung bedeckt ist und dadurch wasser- und ölabweisende Eigenschaften erhalten hat. Dieser Behandlung können sowohl TiO₂ als auch die hier verwendeten farbgebenden Eisenoxide gelbes Eisenoxid, schwarzes Eisenoxid, rotes Eisenoxid oder Gemische unterworfen werden. Durch die Behandlung wird der Aggregationseffekt feinteiliger Metalloxidteilchen wesentlich herabgesetzt; jedoch ist infolge der wasser- und ölabweisenden Eigenschaften die nachfolgende Verarbeitung in bestimmten kosmetischen Produkten nicht ohne weiteres möglich.

Deshalb war es überraschend, daß die Kombination der speziellen ölphase in Verbindung mit den speziellen Pigmenten zu einer stabilen Formulierung führt, wobei erstmals mit Perfluoralkylphosphaten oberflächenbehandelte Pigmente, die wasser- und ölabweisend sind, zu einem stabilen, mit Lichtschutzfaktor 7-10 ausgestatteten und zugleich wasserabweisenden und selbstbräunenden kosmetischen Produkt verarbeitet werden konnten.

Wenn ein Gemisch der Pigmente Titandioxid (P1), gelbes Eisenoxid (P2), schwarzes Eisenoxid (P3) und rotes Eisenoxid (P4) eingesetzt wird, liegt das Verhältnis untereinander P1 : P2 : P3 : P4 vorteilhaft im Bereich von 10 bis 5 : 2 bis 0,50 : 8 bis 0,05 : 1 bis 0,1.

Die Dispergierung dieser physikalischen Filter und Farbkomponenten mit der öligen Phase der Emulsion, die erfindungsgemäß aus Wachsen und Triglyceriden gebildet wird, erfolgt im wesentlichen als Auswahl unter der Vielzahl bekannter Dispergiermittel.

Bekanntlich setzen Dispergiermittel oder Emulgatoren die Grenzflächenspannung zwischen den Phasen z.B. Öl und Wasser herab und erreichen neben der Verringerung der Grenzflächenarbeit auch eine Stabilisierung der gebildeten Emulsion. Da eine Vielzahl von Emulgatoren bekannt ist, siehe z.B. die Aufzählung an kosmetisch interessanten Emulgatoren in der EP-A-0456458, hat der Fachmann sehr große Wahlmöglichkeiten für seine spezielle Emulsion.

Im vorliegenden Fall werden als Dispergiermittel (b1) Cetyl Dimethicone Copolyol und (b2) Cetyl Dimethicone in einem bestimmten Verhältnis zueinander ausgewählt, wobei die in der öligen Phase zu dispergierenden Triglyceride vorzugsweise die der Octan- und/oder Decansäure sind.

Cetyl Dimethicone Dipolyol ist der kosmetisch übliche CTFA-Name für ein Polysiloxan-Polyalkylen-Polyester-Copolymeres mit Polyglycerin-4-isostearat, das auch unter dem Handelsnamen Abil WE 09® bekannt ist. Cetyl Dimethicone ist ein Polyalkyl-Polysiloxan-Copolymeres, das auch unter dem Handelsnamen Abil Wax 9801® bekannt ist.

Als zusätzliches Dispergiermittel kann Cyclomethicone, das unter der Handelsbezeichnung Abil B8839® bekannt ist, in einem Anteil von 1 bis 6 % enthalten sein.

Der Anteil der Dispergiermittel insgesamt beträgt vorzugsweise 4,5 bis 12 Gew-%.

Das erfindungsgemäße Hautbräunungs- und Lichtschutzmittel kann Träger- und Hilfsstoffe umfassen, die aus der Gruppe ausgewählt sind, die aus melaninhaltigen Zubereitungen wie Melanosponges, physikalischen UV-Filtern wie Titandioxid, radikalfangenden und antioxidierenden Substanzen, Feuchthaltemitteln sowie Erweichungsmitteln bestehen.

Wie bereits oben ausgeführt, besteht ein wesentliches Merkmal des erfindungsgemäßen Mittels darin, daß der Gehalt an Feuchthaltemitteln unter 4 Gew-% liegt. Als zusätzliche Feuchthaltemittel können z.B. Aloe vera, Panthenol und andere eingesetzt werden. Das Problem beim Einsatz von Pigmenten besteht darin, daß diese im starken Maße austrocknend auf die Haut wirken, wodurch ein hoher Anteil an Feuchthaltemitteln üblicherweise erforderlich ist. Durch die besondere Kombination der Perfluoralkylphosphat-behandelten Pigmente und der Dispergiermittel im Zusammenhang mit der gewählten öligen Phase ergibt sich überraschend ein wesentlich höherer Feuchtigkeitsgehalt auf der Haut als dies zu erwarten war. Der Gehalt an zusätzlichen Feuchthaltemitteln kann daher vorteilhaft auch unter 2 Gew-% liegen.

Ein weiterer Vorteil der Erfindung liegt im Einsatz physikalischer UV-Filter, wodurch eventuelle Reizwirkungen organischer Filtersubstanzen auf die Haut im wesentlichen ausgeschlossen werden. Die lichtschützende Wirkung der Zusammensetzung liegt im Bereich des Lichtschutzfaktors (LSF) 7 bis 10, vorzugsweise 8 bis 9.

Ein weiterer Vorteil der Erfindung ist darin zu sehen, daß der Selbstbräunungsfilm sehr natürliche Töne erreicht, über einen lange Zeitraum wasserbeständig ist. Dies konnte mit üblichen Wasserbeständigkeits-Tests (nach 5-maligem Eintauchen der Haut in Wasser keine wesentliche Veränderung) nachgewiesen werden. Damit hält auch die Lichtschutzwirkung über einen verlängerten Zeitraum an. Der Film kann mit Seife oder seifenähnlichen Produkten zusammen mit Wasser abgewaschen werden.

Durch Verwendung der gewählten öligen Phase in Form von Wachsen und Triglyceriden kann ein ölfreies Make-up bereitgestellt werden.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische Öle, Butylmyristat, Palmitinsäure usw. Erfindungsgemäß besonders bevorzugt ist jedoch Cyclomethicone, gegebenenfalls im Gemisch mit anderen Erweichungsmitteln.

Die erfindungsgemäße Zusammensetzung ist weich, glatt, nicht klebrig und führt zu einem satin-artigen Hautgefühl und ist nach dem Trocknen nicht färbend für Textilien.

Die Erfindung soll nachstehend durch Beispiele näher erläutert werden. Die angegebenen Teile oder Prozente sind auf das Gewicht bezogen, sofern nichts anderes angegeben ist.

### Beispiel 1

Zur Herstellung eines Hautbräunungsmittels wurden die Phasen C und D miteinander homogenisiert, bis die Pigmente gut dispergiert waren. Die Phase A wurde auf etwa 50 °C erhitzt und dem Pigment-Öl-Gemisch unter starkem Rühren hinzugegeben. Anschließend wurden die restlichen Phasen unter mäßigem Rühren bei Umgebungstemperatur zugesetzt. Alle Angaben sind in Gewichts-%.

| **Phase A** | |
|---|---|
| Deionisiertes Wasser | q.s. |
| Natriumchlorid | 0,2 |

| **Phase B** | |
|---|---|
| Cetyl Dimethicone Dipolyol | 3 |
| Cyclomethicone | 2 |
| Cetyl Dimethicone | 0,5 |
| Caprylic/Capric Triglyceride | 2 |
| Diisopropyl Dimer Dilinoleate | 5 |
| Tocopheryl Acetate | 0,5 |
| Hydrogeniertes Rinzinusöl | 0,1 |
| Bienenwachs | 0,2 |

| **Phase C** | |
|---|---|
| TiO₂ mit Perfluoralkylphosphat | 7,0 |
| Rotes Eisenoxid mit Perfluoralkylphosphat | 0,4 |
| Gelbes Eisenoxid mit Perfluoralkylphosphat | 0,1 |
| Schwarzes Eisenoxid mit Perfluoralkylphosphat | 0,2 |

| **Phase D** | |
|---|---|
| D-Panthenol 50P | 1 |

| **Phase E** | |
|---|---|
| Fragance | 0,2 |
| Konservierungsmittel | 0,2 |

### Beispiel 2

| **Phase A** | |
|---|---|
| Deionisiertes Wasser | q.s. |
| Natriumchlorid | 1 |

| **Phase B** | |
|---|---|
| Cetyl Dimethicone Dipolyol | 4 |
| Cyclomethicone | 3 |
| Cetyl Dimethicone | 0,5 |
| Caprylic/Capric Triglyceride | 3 |
| Diisopropyl Dimer Dilinoleate | 6 |
| Bisabolol | 0,2 |
| Hydrogeniertes Rinzinusöl | 0,3 |
| Bienenwachs | 0,3 |

| **Phase C** | |
|---|---|
| TiO₂ mit Perfluoralkylphosphat | 10,0 |
| Rotes Eisenoxid mit Perfluoralkylphosphat | 0,7 |
| Gelbes Eisenoxid mit Perfluoralkylphosphat | 0,1 |
| Schwarzes Eisenoxid mit Perfluoralkylphosphat | 0,3 |

| **Phase D** | |
|---|---|
| Aloe Vera Gel | 0,1 |

| **Phase E** | |
|---|---|
| D-Panthenol 50P | 1,5 |

| **Phase F** | |
|---|---|
| Fragance | 0,3 |
| Konservierungsmittel | 0,5 |

### Beispiel 3

| **Phase A** | |
|---|---|
| Deionisiertes Wasser | q.s. |
| Natriumchlorid | 1,5 |

| Phase B | |
|---|---|
| Cetyl Dimethicone Dipolyol | 5 |
| Cyclomethicone | 5 |
| Cetyl Dimethicone | 1,5 |
| Caprylic/Capric Triglyceride | 5 |
| Diisopropyl Dimer Dilinoleate | 7 |
| Tocopheryl Acetate | 1 |
| Bisabolol | 0,2 |
| Hydrogeniertes Rizinusöl | 0,4 |
| Bienenwachs | 0,4 |

| **Phase C** | |
|---|---|
| TiO₂ mit Perfluoralkylphosphat | 11 |
| Rotes Eisenoxid mit Perfluoralkylphosphat | 0,9 |
| Gelbes Eisenoxid mit Perfluoralkylphosphat | 0,2 |
| Schwarzes Eisenoxid mit Perfluoralkylphosphat | 0,4 |

| **Phase D** | |
|---|---|
| Aloe Vera Gel | 0,2 |

| **Phase E** | |
|---|---|
| D-Panthenol 50P | 3 |

| **Phase F** | |
|---|---|
| Fragance | 0,5 |
| Konservierungsmittel | 0,8 |

## Patentansprüche

1. Kosmetisches Hautbräunungs- und Lichtschutzmittel, **gekennzeichnet durch** eine O/W-Emulsion, bestehend aus (bezogen auf das Gesamtgewicht der Zusammensetzung)
(a) mit Perfluoralkylphosphaten behandelten anorganischen Pigmenten, wobei die Pigmente aus der Gruppe ausgewählt sind, die aus farbgebenden Eisenoxiden, Titandioxid und deren Gemischen besteht, in einem Anteil von 5 bis 14 Gew-%;
(b) Dispergiermitteln der Gruppe, die wenigstens aus (b1) Cetyl Dimethicone Copolyol und (b2) Cetyl Dimethicone besteht, in einem Anteil im Bereich von 2 bis 10 Gew-% und mit einem Verhältnis von (b1) zu (b2) im Bereich von 15 bis 3 zu 40 bis 8; und
(c) weiteren bekannten Träger- und Hilfsstoffen sowie gegebenenfalls weiteren Dispergiermitteln, wobei der Anteil an zugesetzten Feuchthaltemitteln unter 4 Gew-% liegt.

2. Mittel nach Anspruch 1, **dadurch gekennzeichnet, daß** der Anteil der Pigmente (a) im Bereich von 7 bis 13 Gew-% liegt.

3. Mittel nach Anspruch 1, **dadurch gekennzeichnet, daß** die farbgebenden Eisenoxide gelbes Eisenoxid, schwarzes Eisenoxid, rotes Eisenoxid oder Gemische davon umfassen.

4. Mittel nach Anspruch 1, **dadurch gekennzeichnet, daß** der Anteil der Dispergiermittel insgesamt im Bereich von 4,5 bis 12 Gew-% liegt.

5. Mittel nach Anspruch 1, **dadurch gekennzeichnet, daß** die Träger- und Hilfsstoffe solche umfassen, die aus der Gruppe ausgewählt sind, die aus melaninhaltigen Zubereitungen wie Melanosponges, physikalischen UV-Filtern wie Titandioxid, radikalfangenden und antioxidierenden Substanzen, Feuchthaltemitteln sowie Erweichungsmitteln bestehen.

6. Mittel nach Anspruch 1, **dadurch gekennzeichnet, daß** der Anteil an Feuchthaltemitteln gleich oder kleiner als 2 Gew-% ist.

7. Mittel nach Anspruch 1, **dadurch gekennzeichnet, daß** die ölige Phase von Wachsen und Triglyceriden gebildet wird.

8. Mittel nach einem der Ansprüche 1 bis 7, **dadurch gekennzeichnet, daß** es Radikalfänger und Antioxidationsmittel enthält, die aus der Gruppe ausgewählt sind, bestehend aus α-Tocopherol, Tocopherylacetat, Vitamine A, B, C und Vitamingemische.

## Claims

1. A cosmetic tanning and sunscreen agent, wherein an O/W emulsion is composed of
(a) a share of 5 to 14% by weight of inorganic pigments treated with perfluoroalkyl phosphates, in which the pigments are selected from the group of colored iron oxides, titanium dioxide and mixtures thereof;
(b) dispersants of the group consisting of at least (b1) cetyl dimethicone copolyol and (b2) cetyl dimethicone with a share in the range of 2 to 10% by weight, with the ratio of (b1) to (b2) in the range from 15:3 to 40:8, and
(c) further known carrier and auxiliary agents as well as further dispersants if required, in which the share of moisturizing additives is not to exceed 4% by weight, based on the total weight of the composition.

2. Composition according to Claim 1, wherein the share of pigments (a) is within the range of 7 to 13% by weight.

3. Composition according to Claim 1, wherein the iron oxide colorings consist of yellow, black or red iron oxide or mixtures thereof.

4. Composition according to Claim 1, wherein the total share of dispersants is within the range of 4.5 to 12% by weight.

5. Composition according to Claim 1, wherein the carriers and auxiliary substances are selected from the group of substances consisting of melanin preparations such as melanosponges, physical UV filters such as titanium dioxide, free-radical scavengers and antioxidants, moisturizers and emollients.

6. Composition according to Claim 1, wherein the share of moisturizers is less than or equal to 2 % by weight.

7. Composition according to Claim 1, wherein the oily phase is composed of waxes and triglycerides.

8. Composition according to one of the Claims 1 to 7, **characterized in that** it contains free-radical scavengers and antioxidants selected from the group consisting of α-tocopherol, tocopheryl acetate, vitamins A, B, and C as well as vitamin mixtures.

## Revendications

1. Agent cosmétique pour le bronzage de la peau et pour la photoprotection, **caractérisé par** une émulsion huile dans eau, constitué de (en fonction du poids total de la composition)
(a) pigments inorganiques traités avec des perfluoroalkylphosphates, dans lesquels les pigments sont choisis dans le groupe comprenant les oxydes de fer colorants, le dioxyde de titane et leurs mélanges, en une part de 5 à 14 % en poids ;
(b) agents de dispersion du groupe comprenant au moins (b1) la cétyldiméthicone copolyol et (b2) la cétylediméthicone, en une part comprise entre 2 et 10 % en poids et avec un ratio (b1) sur (b2) compris entre 15 à 3 sur 40 à 8 et
(c) d'autres substances porteuses et additifs ainsi que le cas échéant un agent de dispersion, la part d'agent hydratant à ajouter étant inférieure à 4 % en poids.

2. Agent selon la revendication 1, **caractérisé en ce que** la part des pigments (a) est comprise entre 7 et 13 %.

3. Agent selon la revendication 1, **caractérisé en ce que** les oxydes de fer colorants comprennent l'oxyde de fer jaune, l'oxyde de fer noir, l'oxyde de fer rouge ou leurs mélanges.

4. Agent selon la revendication 1, **caractérisé en ce que** la part d'agent de dispersion est comprise au total entre 4,5 et 12 % en poids.

5. Agent selon la revendication 1, **caractérisé en ce que** les substances porteuses et les additifs comptent ceux qui sont choisis dans le groupe comprenant des préparations contenant de la mélanine comme Mélanosponges, des filtres UV physiques comme le dioxyde de titane, des piégeurs de radicaux et des antioxydants, des agents hydratants et des agents adoucissants.

6. Agent selon la revendication 1, **caractérisé en ce que** la part en agents hydratants est inférieure ou égale à 2 % en poids.

7. Agent selon la revendication 1, **caractérisé en ce que** la phase huileuse est formée de cires et de triglycérides.

8. Agent selon l'une des revendications 1 à 7, **caractérisé en ce qu'**il contient un piégeur de radicaux et un anti-oxydant choisis dans le groupe comprenant l'α-tocophérol, l'acétate de tocophéryle, les vitamines A, B, C et les mélanges de vitamines.
